(19) Europäisches Patentamt
European Patent Office
Office européen des brevets

(11) **EP 4 635 563 A1**

(12) **EUROPEAN PATENT APPLICATION**

(43) Date of publication:
**22.10.2025  Bulletin 2025/43**

(21) Application number: **25169825.4**

(22) Date of filing: **10.04.2025**

(51) International Patent Classification (IPC):
***A61N 5/10*** *(2006.01)*

(52) Cooperative Patent Classification (CPC):
**A61N 5/1031**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority:  **17.04.2024  US 202418637599**

(71) Applicant: **Siemens Healthineers International AG 6312 Steinhausen (CH)**

(72) Inventors:
• **CZEIZLER, Elena**
  **00390 Helsinki (FI)**
• **CORDERO MARCOS, Maria**
  **02620 Espoo (FI)**
• **KUUSELA, Esa**
  **02320 Espoo (FI)**

(74) Representative: **Mathisen & Macara LLP Charta House 30-38 Church Street Staines-upon-Thames TW18 4EP (GB)**

(54) **METHOD AND APPARATUS TO FACILITATE OPTIMIZING A RADIATION TREATMENT PLAN**

(57)     A control circuit can access 202 predicted three-dimensional radiation dose distribution information and optimize 204 a radiation treatment plan as a function, at least in part, of the predicted three-dimensional radiation dose distribution information to thereby prompt optimization towards the predicted three-dimensional radiation dose distribution information. By one approach, these teachings will support using the predicted three-dimensional radiation dose distribution information as an optimization constraint.

FIG. 2

## Description

TECHNICAL FIELD

These teachings relate generally to treating a patient's planning target volume with energy pursuant to an energy-based treatment plan and more particularly to optimizing an energy-based treatment plan.

BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Dose prediction can help to identify the type of dose distribution one may expect a current patient to experience based on previously-planned cases for other patients. Typically, a predicted dose distribution only gives an idea of what might be achievable. It usually remains up to the user to do a series of planning attempts to get as close as possible to a given desired result. Such an approach tends to be laborious, highly time consuming, and error prone.

SUMMARY

In one aspect the present invention provides a method to facilitate optimizing a radiation treatment plan as defined in claim 1. Optional features are specified in the dependent claims.

In another aspect the present invention provides an apparatus to facilitate optimizing a radiation treatment plan as defined in claim 11. Optional features are specified in the dependent claims.

BRIEF DESCRIPTION OF DRAWINGS

The above needs are at least partially met through provision of the method and apparatus to facilitate optimizing a radiation treatment plan described in the following detailed description, particularly when studied in conjunction with the drawings, wherein:

FIG. 1 comprises a block diagram as configured in accordance with various embodiments of these teachings; and
FIG. 2 comprises a flow diagram as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

DETAILED DESCRIPTION

Generally speaking, pursuant to these various embodiments, a control circuit can access predicted three-dimensional radiation dose distribution information and optimize a radiation treatment plan as a function, at least in part, of the predicted three-dimensional radiation dose distribution information to thereby prompt optimization towards the predicted three-dimensional radiation dose distribution information.

By one approach, these teachings will support using the predicted three-dimensional radiation dose distribution information as an optimization constraint. By one approach, that optimization constraint can comprise an optimization cost function that can be formed using at least part of the predicted three-dimensional radiation dose distribution information as a goal value for at least some three-dimensional-voxels.

If desired, these teachings will accommodate using other information to determine at least one weight and/or at least one functional form of individual voxel costs. The aforementioned "other information" can comprise, by one approach, information that corresponds to non-three-dimensional clinical goals that are related to at least one patient target volume and/or at least one patient organ structure, patient images, patient geometry information, spatial definitions of a patient target volume, a non-targeted patient organ and/or patient body structures, field geometry information, and/or radiation treatment platform information.

These teachings are highly practical and flexible in practice and will accommodate any of a variety of supplemental and/or modified features. As one example in these regards, the foregoing optimization can further comprise using an optimization cost function having at least one normalization factor. At least one such normalization factor can correspond, for example, to the accuracy of the predicted three-dimensional radiation dose distribution information.

As another example of the flexibility of these teachings, the foregoing approaches can also accommodate, for example, accessing clinical goals for the patient and then optimizing the radiation treatment plan as a function, at least in part, of both the predicted three-dimensional radiation dose distribution information and information pertaining to those clinical goals. Those clinical goals may comprise, for example, at least one of a one-dimensional and a two-dimensional constraint. When so using one or more clinical goals, the foregoing optimization may include, for example, forming at least one optimization cost function having weights that are determined as a function, at least in part, of complying with at least one clinical goal.

So configured, and by one illustrative approach, these teachings will support taking a predicted three-dimensional dose matrix as an input to an optimization algorithm to guide and drive that algorithm, such that the optimizer will converge towards the predicted dose for a target volume(s) in a straightforward manner while likely avoiding a need for multiple planning attempts (while also, if desired, simultaneously following guidance from the clinical goals).

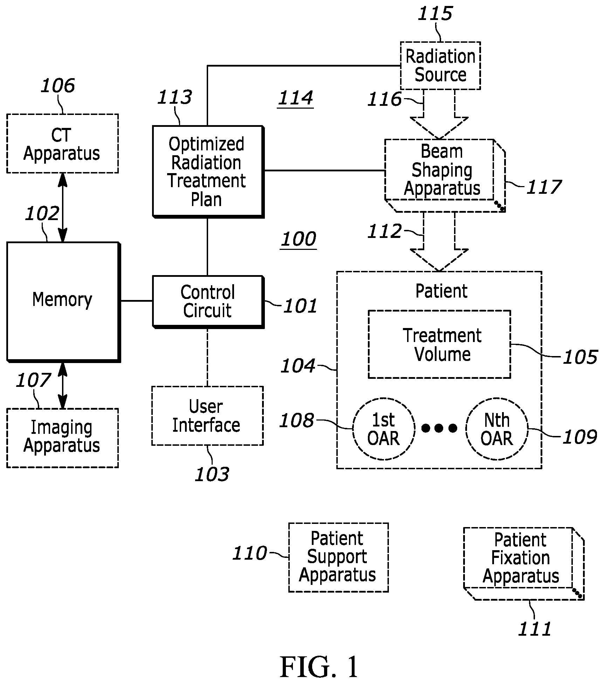

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as clinical goals, optimization information for a particular patient, and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM)).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

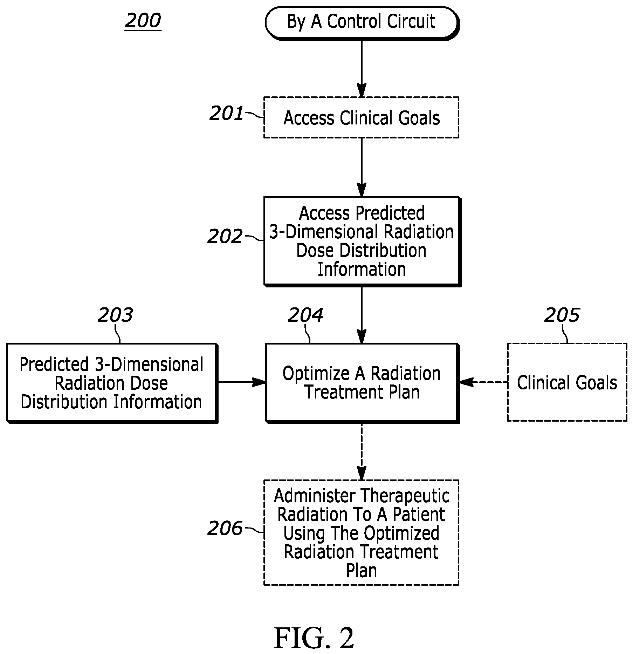

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient 104 with therapeutic radiation 112 using a particular radiation treatment platform 114 per that optimized radiation treatment plan 113.

At optional block 201, this process 200 provides for optionally accessing clinical goals for a given patient. By one approach, this may comprise the control circuit 101 accessing the aforementioned memory 102. Clinical goals are the treatment goals being prescribed by, for example, an attending oncologist. Examples of clinical goals include, but are not limited to, goals regarding the dose distributions to be achieved with respect to a target volume, one or more organs-at-risk in the vicinity of the target volume, or other specified or unspecified normal tissues. By their very nature, clinical goals are typically agnostic with respect to what physical radiation treatment platform serves to administer the radiation. These teachings will accommodate, for example, clinical goals comprising either of a one-dimensional or a two-dimensional constraint.

At block 202, the control circuit 101 accesses predicted three-dimensional radiation dose distribution information. Various approaches are known in the art regarding how to formulate such a prediction. By one approach, for example, these teachings will accommodate predicting a particular dose distribution for a given patient by referring to historical dose distribution information representing other previous patients. Such a prediction can be formed, for example, using a machine learning model that uses such information from existing, previously-administered plans to make a corresponding dose distribution prediction for the current patient.

This predicted three-dimensional radiation dose distribution information 203 is then used by the control circuit 101, at block 204, to optimize a radiation treatment plan as a function, at least in part, of that predicted three-dimensional radiation dose distribution information to thereby prompt optimization towards the predicted three-dimensional radiation dose distribution information 203. By one approach, using the predicted three-dimensional radiation dose distribution information 203 in this way can comprise using the predicted three-dimensional radiation dose distribution information 203 as an optimization constraint. Generally speaking, an optimization constraint serves to establish a condition or set of conditions that an optimization solution must satisfy in order to be considered viable.

By one approach, these teachings will accommodate forming at least one optimization cost function using at least part of the predicted three-dimensional radiation dose distribution information 203 as a goal value for at least some three-dimensional-voxels that correspond to a patient volume of interest (i.e., a targeted volume or a particular volume to be spared from radiation).

By one approach, forming at least one optimization cost function using at least part of the predicted three-dimensional radiation dose distribution information 203 as a goal value for at least some three-dimensional voxels can further comprise using other information (beyond the predicted three-dimensional radiation dose distribution information 203) to determine at least one weight and/or at least one functional form of individual voxel costs. For example, at least one optimization cost function having one or more corresponding weights can be formed that are determined as a function, at least in part, of complying with at least one clinical goal. Examples of potentially useful "other information" include, but are not limited to, (a) information corresponding to non-three-dimensional clinical goals related to at least one patient target volume and/or at least one patient organ structure, (b) patient images (including, for example, segmented patient images), (c) patient geometry information, (d) spatial definitions of a patient target volume, a non-targeted patient organ, and/or patient body structures, (e) field geometry information, and/or (f) radiation treatment platform 114 information.

By another approach, in lieu of the foregoing or in combination therewith, these teachings will accommodate optimizing the radiation treatment plan as a function, at least in part, of the predicted three-dimensional radiation dose distribution information 203 by using an optimization cost function having at least one normalization factor. Such a normalization factor can correspond, for example, to the accuracy of the predicted three-dimensional radiation dose distribution information 203.

As noted above, by one optional approach this process 200 can provide for accessing clinical goals. In such a case, the resultant accessed clinical goals 205 can also be utilized when optimizing the radiation treatment plan. In particular, and by example, optimizing the radiation treatment plan as a function, at least in part, of the predicted three-dimensional radiation dose distribution information 203 can further comprise optimizing the radiation treatment plan as a function, at least in part, of the predicted three-dimensional radiation dose distribution information 203 and information pertaining to the clinical goals 205.

So configured, these teachings will support taking a predicted three-dimensional dose matrix as an input to an optimization algorithm to guide and drive that algorithm, so that the optimizer will converge towards that predicted dose for the corresponding volume in a straightforward manner without the need for multiple planning attempts, while simultaneously also taking guidance from the clinical goals as well. It may also be noted that although the predicted three-dimensional radiation dose distribution information 203 may (likely) comprise and represent information for a (potentially) large number of patients (none of whom may be the current patient), that predicted dose distribution can nevertheless serve as a useful optimization constraint when optimizing a radiation treatment plan for a single particular current patient.

As illustrated at optional block 206, the resultant optimized radiation treatment plan 113 can then be used to administer therapeutic radiation 112 to the given patient 104.

Further details that comport with these teachings will now be presented. It will be understood that the specific details of these examples are intended to serve an illustrative purpose and are not intended to suggest any particular limitations with respect to these teachings.

By one approach, these teachings will provide for inputting to an optimizer a predicted three-dimensional dose (which can be used as a three-dimensional optimization constraint) along with clinical goal information (which can be used as one-dimensional and/or two-dimensional optimization constraints) related to target volumes and protected volumes. Additionally, the optimizer can also receive as input standard optimization information such as a computed tomography image stack (or similar patient geometry information), spatial definitions for one or more target volumes, organs-at-risk, and/or body structures, field geometry, and/or information that characterizes one or more components of the radiation treatment platform 114.

An optimization cost function can be constructed using the aforementioned predicted three-dimensional radiation dose distribution information 203 as a goal value for each three-dimensional-voxel, while at least some of the other information can be used to determine the weight(s) and/or a functional form of individual voxel costs. The functional form of the cost function related to the predicted 3d dose can be, by one illustrative approach:

$$C_{pred} = \sum_{i \in V} w_i f_i \left( \frac{d_i - d_i^*}{\tilde{d}_i} \right)$$

where $d_i$ is the dose at voxel $i$, and $d_i^*$ is the predicted dose in the same voxel. The difference in this example is normalized with $\tilde{d}_i$ which is related to the accuracy of the prediction and $w_i$ is a weight of the voxel. Note that the functional form $f_i$ might be different for different voxels if desired.

The normalization factor $\tilde{d}_i$ can depend on the accuracy of the three-dimensional dose prediction by referring, for example, to a corresponding confidence matrix. By one approach, a confidence matrix can be output by the predicted three-dimensional radiation dose distribution information 203 prediction algorithm. Confidence can be assessed in a variety of ways. By one approach, confidence can be assessed as a function of distance to a given target. By another approach, in lieu of the foregoing or in combination therewith, confidence can be assessed as a function of distance to both a target volume as well as significant organs-at-risk (for example, in some cases a lower dose can always be observed in the vicinity of the spinal cord, thus leading to higher confidence in this case than perhaps in other areas of soft tissue). And by yet another approach, confidence can be assessed as a function of whether a given voxel in question belongs to a target volume, an organ-at-risk, or unlabeled normal tissue.

By one approach, voxel weights $w_i$ can be used to take into account the clinical information that is available. For example, it could be beneficial to use higher weights for voxels that belong to critical organs, with the weight increasing when the volume of the organ is decreased. The weight could be, for example, related to the inverse volume of the smallest organ to which the voxel belongs.

By one approach, another factor in $w_i$ could be calculated based on satisfaction of the clinical goals. For example, any violated clinical goal could lead to an increased weight in the cost of those voxels that are currently contributing to the failure of that goal. As one illustrative example in these regards, when $d_{max}$ for a certain organ is beyond a set value, all voxels belonging to that organ and having a current dose larger than the set value can be accorded a higher weight. As another illustrative example, when the mean dose of another organ is too high, all voxels belonging to that organ can be accorded a higher weight, since all voxels are contributing to the mean dose.

By one approach, it might be decided that a given organ-at-risk can be sacrificed (via, for example, user interaction or some other additional input) and the corresponding weight can be lowered for all the voxels belonging to the sacrificed organ-at-risk.

By one approach, information related to the clinical goals can also be taken into account as added terms to a cost function that is related to one-dimensional and/or two-dimensional optimization constraints (for example, as a separate term in addition to the aforementioned three-dimensional dose-related optimization cost).

By yet another approach, the functional form of the voxel cost ($f_i$) can be related to the organ to which the voxel belongs. When a given voxel does not belong to any targeted structure, it might be beneficial to only penalize voxel doses that are higher than the predicted dose. Inside a target structure the functional form can be such that any deviation from the predicted dose level could be penalized. If desired, these teachings will also accommodate using a functional form that has a residual cost even when the goal level has been reached (such as, for example, an exponential function) to thereby maintain an optimization pressure or bias to reduce the dose in normal untargeted tissue even when a predicted dose level is achieved.

By one approach, these teachings will accommodate calculating the cost for a spatial constraint based on predicted three-dimensional radiation dose distribution information 203 as a sum of the cost in each voxel. For each voxel, the cost can be defined as the squared distance of the current optimized dose to the desired (i.e., predicted) dose, multiplied by the confidence on that voxel as well as the overall spatial constraint weight.

Those skilled in the art will recognize that a wide variety of modifications, alterations, and combinations can be made with respect to the above-described embodiments without departing from the scope of the invention. As but one example in these regards, point constraints can be input by a user to indicate patient areas in which the predicted dose did not present satisfactory results (for example, as regards target homogeneity). This additional point constraint could be entered directly by the planner or could be automatically derived and set from the three-dimensional dose to add more pressure in certain areas. In other application settings, the user could input these results in a three-dimensional optimization matrix by painting small areas in the three-dimensional dose matrix with lower or higher doses. It will therefore be understood that such modifications, alterations, and combinations are to be viewed as being within the ambit of the inventive concept.

Further aspects of the invention are provided by the subject matter of the following clauses:

Clause 1. A method to facilitate optimizing a radiation treatment plan, comprising: by a control circuit: accessing predicted three-dimensional radiation dose distribution information; optimizing the radiation treatment plan as a function, at least in part, of the predicted three-dimensional radiation dose distribution information to thereby prompt optimization towards the predicted three-dimensional radiation dose distribution information.

Clause 2. The method of any of the foregoing method clauses wherein optimizing the radiation treatment plan as a function, at least in part, of the predicted three-dimensional radiation dose distribution information comprises, at least in part by using the predicted three-dimensional radiation dose distribution information as an optimization constraint.

Clause 3. The method of any of the foregoing method clauses further comprising: by the control circuit: accessing clinical goals; and wherein optimizing the radiation treatment plan as a function, at least in part, of the predicted three-dimensional radiation dose distribution information further comprises optimizing the radiation treatment plan as a function, at least in part, of the predicted three-dimensional radiation dose distribution information and information pertaining to the clinical goals.

Clause 4. The method of any of the foregoing method clauses wherein the clinical goals include, at least in part, at least one of a one-dimensional and a two-dimensional constraint.

Clause 5. The method of any of the foregoing method clauses wherein optimizing the radiation treatment plan as a function, at least in part, of the predicted three-dimensional radiation dose distribution information further comprises forming at least one optimization cost function using at least part of the predicted three-dimensional radiation dose distribution information as a goal value for at least some three-dimensional-voxels.

Clause 6. The method of any of the foregoing method clauses wherein forming at least one optimization cost function using at least part of the predicted three-dimensional radiation dose distribution information as a goal value for at least some three-dimensional voxels further comprises using other information to determine at least one weight and/or at least one functional form of individual voxel costs.

Clause 7. The method of any of the foregoing method clauses wherein the other information includes at least one of: information corresponding to non-three-dimensional clinical goals related to at least one patient target volume and/or at least one patient organ structure; patient images; patient geometry information; spatial definitions of a patient target volume, a non-targeted patient organ, and/or patient body structures; field geometry information; and/or radiation treatment platform information.

Clause 8. The method of any of the foregoing method clauses wherein optimizing the radiation treatment plan as a function, at least in part, of the predicted three-dimensional radiation dose distribution information further comprises using an optimization cost function having at least one normalization factor.

Clause 9. The method of any of the foregoing method clauses wherein the at least one normalization factor corresponds to accuracy of the predicted three-dimensional radiation dose distribution information.

Clause 10. The method of any of the foregoing method clauses wherein optimizing the radiation treatment plan as a function, at least in part, of the predicted three-dimensional radiation dose distribution information further comprises forming at least one optimization cost function having weights that are determined as a function, at least in part, of complying with at least one clinical goal.

Clause 11. An apparatus to facilitate optimizing a radiation treatment plan, comprising: a control circuit configured to: access predicted three-dimensional radiation dose distribution information; optimize the radiation treatment plan as a function, at least in part, of the predicted three-dimensional radiation dose distribution information to thereby prompt optimization towards the predicted three-dimensional radiation dose distribution information.

Clause 12. The apparatus of any of the foregoing apparatus clauses wherein the control circuit is configured to optimize the radiation treatment plan as a function, at least in part, of the predicted three-dimensional radiation dose distribution information, at least in part, by using the predicted three-dimensional radiation dose distribution information as an optimization constraint.

Clause 13. The apparatus of any of the foregoing apparatus clauses wherein the control circuit is further configured to: access clinical goals; and wherein the control circuit is configured to optimize the radiation treatment plan as a function, at least in part, of the predicted three-dimensional radiation dose distribution information by optimizing the radiation treatment plan as a function, at least in part, of the predicted three-dimensional radiation dose distribution information and information pertaining to the clinical goals.

Clause 14. The apparatus of any of the foregoing apparatus clauses wherein the clinical goals include, at least in part, at least one of a one-dimensional and a two-dimensional constraint.

Clause 15. The apparatus of any of the foregoing apparatus clauses wherein the control circuit is configured to optimize the radiation treatment plan as a function, at least in part, of the predicted three-dimensional radiation dose distribution information by forming at least one optimization cost function using at least part of the predicted three-dimensional radiation dose distribution information as a goal value for at least some three-dimensional-voxels.

Clause 16. The apparatus of any of the foregoing apparatus clauses wherein the control circuit is configured to form at least one optimization cost function using at least part of the predicted three-dimensional radiation dose distribution information as a goal value for at least some three-dimensional voxels by using other information to determine at least one weight and/or at least one functional form of individual voxel costs.

Clause 17. The apparatus of any of the foregoing apparatus clauses wherein the other information includes at least one of: information corresponding to non-three-dimensional clinical goals related to at least one patient target volume and/or at least one patient organ structure; patient images; patient geometry information; spatial definitions of a patient target volume, a non-targeted patient organ, and/or patient body structures; field geometry information; and/or radiation treatment platform information.

Clause 18. The apparatus of any of the foregoing apparatus clauses wherein the control circuit is configured to optimize the radiation treatment plan as a function, at least in part, of the predicted three-dimensional radiation dose distribution information by using an optimization cost function having at least one normalization factor.

Clause 19. The apparatus of any of the foregoing apparatus clauses wherein the at least one normalization factor corresponds to accuracy of the predicted three-dimensional radiation dose distribution information.

Clause 20. The apparatus of any of the foregoing apparatus clauses wherein the control circuit is configured to optimize the radiation treatment plan as a function, at least in part, of the predicted three-dimensional radiation dose distribution information by forming at least one optimization cost function having weights that are determined as a function, at least in part, of complying with at least one clinical goal.

**Claims**

1. A method to facilitate optimizing a radiation treatment plan, comprising:
   by a control circuit:

   accessing predicted three-dimensional radiation dose distribution information;
   optimizing the radiation treatment plan as a function, at least in part, of the predicted three-dimensional radiation dose distribution information to thereby prompt optimization towards the predicted three-dimensional radiation dose distribution information.

2. The method of claim 1 wherein optimizing the radiation treatment plan as a function, at least in part, of the predicted three-dimensional radiation dose distribution information comprises, at least in part by using the predicted three-dimensional radiation dose distribution information as an optimization constraint.

3. The method of claim 1 or 2 further comprising:

   by the control circuit:
   accessing clinical goals;
   and wherein optimizing the radiation treatment plan as a function, at least in part, of the predicted three-dimensional radiation dose distribution information further comprises optimizing the radiation treatment plan as a function, at least in part, of the predicted three-dimensional radiation dose distribution information and information pertaining to the clinical goals.

4. The method of claim 3 wherein the clinical goals include, at least in part, at least one of a one-dimensional and a two-dimensional constraint.

5. The method of any one of claims 1 to 4 wherein optimizing the radiation treatment plan as a function, at least in part, of the predicted three-dimensional radiation dose distribution information further comprises forming at least one optimization cost function using at least part of the predicted three-dimensional radiation dose distribution information as a goal value for at least some three-dimensional-voxels.

6. The method of claim 5 wherein forming at least one optimization cost function using at least part of the predicted three-dimensional radiation dose distribution information as a goal value for at least some three-dimensional voxels further comprises using other information to determine at least one weight and/or at least one functional form of individual voxel costs.

7. The method of claim 6 wherein the other information includes at least one of:

   information corresponding to non-three-dimensional clinical goals related to at least one patient target volume and/or at least one patient organ structure;
   patient images;
   patient geometry information;
   spatial definitions of a patient target volume, a non-targeted patient organ, and/or patient body structures;
   field geometry information; and/or
   radiation treatment platform information.

8. The method of any one of claims 1 to 7 wherein optimizing the radiation treatment plan as a function, at least in part, of the predicted three-dimensional radiation dose distribution information further comprises using an optimization cost function having at least one normalization factor.

9. The method of claim 8 wherein the at least one normalization factor corresponds to accuracy of the predicted three-dimensional radiation dose distribution information.

10. The method of any one of claims 1 to 9 wherein optimizing the radiation treatment plan as a function, at least in part, of the predicted three-dimensional radiation dose distribution information further comprises forming at least one optimization cost function having weights that are determined as a function, at least in part, of complying with at least one clinical goal.

11. An apparatus to facilitate optimizing a radiation treatment plan, comprising:
a control circuit configured to:

access predicted three-dimensional radiation dose distribution information;
optimize the radiation treatment plan as a function, at least in part, of the predicted three-dimensional radiation dose distribution information to thereby prompt optimization towards the predicted three-dimensional radiation dose distribution information.

12. The apparatus of claim 11 wherein the control circuit is configured to optimize the radiation treatment plan as a function, at least in part, of the predicted three-dimensional radiation dose distribution information, at least in part, by using the predicted three-dimensional radiation dose distribution information as an optimization constraint.

13. The apparatus of claim 11 or 12 wherein the control circuit is further configured to:
access clinical goals;
and wherein the control circuit is configured to optimize the radiation treatment plan as a function, at least in part, of the predicted three-dimensional radiation dose distribution information by optimizing the radiation treatment plan as a function, at least in part, of the predicted three-dimensional radiation dose distribution information and information pertaining to the clinical goals.

14. The apparatus of claim 13 wherein the clinical goals include, at least in part, at least one of a one-dimensional and a two-dimensional constraint.

15. The apparatus of any one of claims 11 to 14 wherein the control circuit is configured to perform the method of any one of claims 5 to 10.

FIG. 1

FIG. 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/196533 A1 (MEMORIAL SLOAN KETTERING CANCER CENTER [US] ET AL.) 12 October 2023 (2023-10-12) * paragraphs [0033], [0040], [0058], [0063], [0064] * | 1-15 | INV. A61N5/10 |
| X | CN 110 327 554 A (UNIV SOUTHERN MEDICAL) 15 October 2019 (2019-10-15) * claims 1,4,5; example 3 * | 1-15 | |
| X | US 2020/155868 A1 (YUAN YADING [US] ET AL) 21 May 2020 (2020-05-21) * paragraphs [0021], [0024], [0050], [0070], [0071]; figure 1; table V * | 1-15 | |
| X | CN 110 124 214 A (UNIV SOUTHERN MEDICAL) 16 August 2019 (2019-08-16) * claims 1, 7 * | 1-15 | |

TECHNICAL FIELDS
SEARCHED       (IPC)

A61N

The present search report has been drawn up for all claims

| Place of search | Date of completion of the search | Examiner |
|---|---|---|
| Munich | 7 August 2025 | Link, Tatiana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 9825

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2025

| Patent document cited in search report | Publication date | Patent family member(s) | | Publication date |
|---|---|---|---|---|
| WO 2023196533 A1 | 12-10-2023 | US | 2025229104 A1 | 17-07-2025 |
| | | WO | 2023196533 A1 | 12-10-2023 |
| CN 110327554 A | 15-10-2019 | NONE | | |
| US 2020155868 A1 | 21-05-2020 | EP | 3661597 A1 | 10-06-2020 |
| | | US | 2020155868 A1 | 21-05-2020 |
| | | WO | 2019027924 A1 | 07-02-2019 |
| CN 110124214 A | 16-08-2019 | NONE | | |

EPO FORM P0459

For more details about this annex : see Official Journal of the European Patent Office, No. 12/82